# EUROPEAN PATENT APPLICATION

(11) **EP 2 371 414 A1**
(43) Date of publication of application: **05.10.2011**
(21) Application number: 09831978.3
(22) Date of filing: 11.12.2009
(51) Int. Cl.: A61M 25/00

(54) **MEDICAL TUBE**

(30) Priority: 11.12.2008 JP 2008316250
(71) Applicant: Kaneka Corporation, Osaka-shi, Osaka 530-8288 (JP)
(72) Inventor: SAKATA Akitoshi, Settsu-shi Osaka 566-0072 (JP); NAKAO Fumihiko, Settsu-shi Osaka 566-0072 (JP)
(74) Representative: TBK
(86) International application number: PCT/JP2009/070782
(87) International publication number: WO 2010/067875

(57) **Abstract**

Provided is a medical tube which is flexible and achieves good kink resistant properties and high tensile strength at the same time. Even when connected to another tube, the medical tube maintains the above-mentioned kink resistant properties and tensile strength. A medical tube is provided with at least one coil layer, a first outer layer provided outside the coil layer, and a second outer layer provided outside the first outer layer. The melting point of the material which forms the second outer layer is lower than the melting point of the material forming the first outer layer. The outer surface of the coil layer and the inner surface of the first outer layer are in contact with and affixed to each other in a slidable state.

## Description

### Technical Field

The present invention relates to a medical tube superior in flexibility and tensile strength and a medical assembly and a catheter produced by using the same.

### Background Art

Medical practices such as administration or injection of medicines, embolic materials, contrast media, and others and suction of thrombus and others through a catheter percutaneously inserted into blood vessel and guided into an organ such as brain, heart or abdomen have been implemented traditionally. Recently, in progress of medicine, there exists a need for injection of medicines, embolic materials, contrast media, and others into finer peripheral blood vessels and also for suction of thrombus and others therefrom. There is also a need for convenience in catheter operation. Examples of the operational convenience include efficiency of conveying the pressurization force applied by a surgeon to the distal end of catheter (pushability), efficiency of guiding the catheter through fine bent peripheral blood vessels, resistance of the catheter to crimping in the bent or curved region of blood vessel (kink resistance), and the like. A reinforcing layer in a braid or coil structure may be formed in the catheter to assure the favorable kink resistance and guiding efficiency in peripheral blood vessels.

The inventors have found that the coil structure shows its favorable properties particularly in the flexible region at the distal end side of the catheter by its superior kink resistance and lumen-retaining efficiency when the catheter is bent significantly. However, there was also found a problem that, when the reinforcing layer is more flexible, the materials for the internal layer and/or outer layer other than the coil layer, which are weaker to tension, are easily broken. Thus, the outer layer should be thickened and it was technically difficult to reduce the thickness of the layer. Although the distal region of catheter demands flexibility particularly, use of a soft resin, which has a small tensile strength, caused a concern about deterioration in tensile strength of the catheter.

Proposed as a method of improving the tensile strength of catheter shaft is a method of using an axially directed member in the catheter longitudinal direction. For example, Patent Document 1 describes a vascular catheter further having an axially directed member extending along the reinforcing layer of a braid. The axially directed member, when used, prevents expansion of the shaft. In addition, the axially directed member has a structure in which it is not fixed to any polymer layer in contact with the braid. However, it is indeed possible by the method to prevent expansion in the axial direction, but the wire strength of the axially directed member should be raised for resistance to higher tensile force, which may possibly lead to anisotropy in flexural rigidity. Alternatively, an axially directed member, if formed in a helically-wound coil structure, may give projections along the lengthwise direction of the catheter, as it is concerned in the literature above.

Also proposed as a method of improving the tensile strength of a coil structure is a method of forming a braided structure outside the coil structure. For example, in Patent Document 2, a metal flat-plate densely wound coil is covered with a metal flat-wire braided and additionally a resin-coating layer formed thereon. Both resistance to the compressive during bending by the coil structure and resistance to the tensile force by the fabric structure are desired. However, if the thickness or width of wire constituting the braid is increased for assurance of high tensile force, the resistance to the compressive force during bending by the coil structure declines, and thus, it is difficult to apply it to the distal end side of a catheter that demands flexibility and also resistance to high tensile force.

### Citation List

### Patent Literature

Patent Document 1: JP-A No. 2002-535049
Patent Document 2: JP No. 2541872

### Summary of Invention

### Technical Problem

An object of the present invention is to provide a medical tube flexible and superior both in kink resistance and tensile strength, and a medical tube that retains the properties even when the medical tube is thermally bonded to another tube.

### Solution to Problem

After intensive studies to solve the problems above, the inventors have made an invention, which provides:
(1) a medical tube comprising at least one coil layer, a first outer layer formed outside the coil layer, and a second outer layer formed outside the first outer layer, wherein the material constituting the second outer layer has a melting point lower than that of the material constituting the first outer layer and the external surface of the coil layer and the internal surface of the first outer layer are in contact with each other in a slidable state. A flexible and high-tensile-strength medical tube is obtained in this way.

The state where "the external surface of the coil layer and the internal surface of the first outer layer are in contact with each other in a slidable state," as used in the present invention, means that the external surface of the coil layer and the internal surface of the first outer layer are in contact with and fixed to each other, in such a manner that they do not slide from each other for example by frictional force when no stress is applied to the medical tube, but the coil layer and the first outer layer slide independently of each other, when stress is applied to the medical tube.

The invention also provides (2) the medical tube, wherein the external surface of the coil layer and the internal surface of the first outer layer are in contact with each other in a slidable state, at a rate of at least of 0.5 or more with respect to the entire coil layer, and
(3) the medical tube, wherein the first outer layer is made of a thermoplastic resin having a Shore D hardness of 50 D or more.

It also provides (4) the medical tube, wherein the coil layer has an internal layer inside. In this way, a medical tube higher in tensile strength that has a lumen superior in lubricity is obtained.

It also provides (5) the medical tube, wherein the coil layer is configured with a coil having space between neighboring wires, and
(6) the medical tube, wherein the distance between the coil wires in the longitudinal direction of the medical tube is the same as or longer than the width of the coil wire. In this way, a medical tube particularly superior in flexibility is obtained.

It also provides (7) the medical tube, wherein the first outer layer is oriented in the axial direction.

It also provides (8) the medical tube, wherein an intermediate layer of a flexible material is formed in space between the coil wires. It is possible in this way to produce a favorable medical tube easily.

It also provides (9) a medical assembly, comprising the medical tube (first medical tube) and a second tube containing at least partially a resin material having a melting point lower than that of the material constituting the first outer layer of the first medical tube, wherein the region of the resin material in the second tube is thermally bonded to the first tube. In this way, a medical assembly flexible, kink-resistant, and superior in tensile strength is obtained.

It provides (10) the medical assembly, wherein the region of the resin material in the second tube is placed on the external surface of the second tube. In this way, a medical assembly having two lumens flexible, kink-resistant, and superior in tensile strength is obtained.

It also provides (11) a catheter, at least partially comprising the medical tube or the medical assembly, and
(12) the catheter, wherein the medical tube or the medical assembly is formed in the distal region thereof.

It also provides (13) a thrombus aspiration catheter, wherein the medical tube or the medical assembly is applied at least in a region. In this way, a catheter, specifically a thrombus aspiration catheter, flexible, kink-resistant, and superior in tensile strength is obtained.

It also provides (14) a method of producing a medical assembly containing the medical tube (first medical tube) and a second tube containing at least partially a resin material having a melting point lower than that of the material constituting the first outer layer of the first medical tube, characterized in that the region of the resin material in the second tube is thermally bonded to the first tube at a temperature lower than the melting point of the material constituting the first outer layer of the first medical tube and higher than the melting point of the material constituting the second outer layer. In this way, a method of producing a medical assembly flexible, kink-resistant, and superior in tensile strength is provided.

It also provides (15) the method of producing a medical assembly, wherein the region of the resin material in the second tube, which is placed on the external surface of the second tube, is thermally bonded to the first tube.

### Advantageous Effects of Invention

According to the present invention, it is possible to provide a flexible medical tube superior both in kink resistance and tensile strength. It is also possible to provide a medical tube retaining the properties described above, even when it is thermally bonded to another tube. As a result, it can be used effectively as a component of various catheters.

### Brief Description of the Drawings

Figure 1 is a cross-sectional view in the side wall direction of the medical tube according to the present invention in an embodiment.
Figure 2 is a cross-sectional view in the side wall direction of the medical tube according to the present invention in another embodiment.
Figure 3(a) is a schematic cross-sectional view in the side wall direction of a conventional medical tube when no stress is applied thereto.
Figure 3(b) is a schematic cross-sectional view in the side wall direction of a conventional medical tube when stress (tensile stress in the tube longitudinal direction) is applied thereto.
Figure 4(a) a schematic cross-sectional view in the side wall direction of a conventional medical tube when no stress is applied thereto.
Figure 4(b) is a schematic cross-sectional view in the side wall direction of a conventional medical tube when stress (bending stress) is applied thereto.
Figure 5(a) is a schematic cross-sectional view in the side wall direction of the medical tube according to the present invention when no stress is applied thereto.
Figure 5(b) is a schematic cross-sectional view in the side wall direction of the medical tube according to the present invention when stress (tensile stress in the tube longitudinal direction) is applied thereto.
Figure 6(a) is a schematic cross-sectional view in the side wall direction of the medical tube according to the present invention when no stress is applied thereto.
Figure 6(b) is a schematic cross-sectional view in the side wall direction of the medical tube according to the present invention when stress (bending stress) is applied thereto.
Figure 7(a) is a schematic cross-sectional view in the side wall direction of the thrombus aspiration catheter according to the present invention in an embodiment.
Figure 7(b) is a schematic view illustrating the X-X cross section shown in Figure 7(a).
Figure 8(a) is an expanded schematic cross-sectional view in the side wall direction of the distal region of the thrombus aspiration catheter shown in Figure 7(a).
Figure 8 (b) is an expanded schematic cross-sectional view in the side wall direction of the distal region of the thrombus aspiration catheter according to the present invention in another embodiment.
Figure 9 is a schematic cross-sectional view in the side wall direction of the medical assembly obtained in an embodiment of the production method according to the present invention.

### Description of Embodiments

The present invention relates to a medical tube comprising at least one coil layer, a first outer layer formed outside the coil layer and a second outer layer formed outside the first outer layer, wherein the material constituting the second outer layer has a melting point lower than that of the material constituting the first outer layer and the external surface of the coil layer and the internal surface of the first outer layer are in contact with each other in a slidable state. It is possible in this way to provide a medical tube that is superior in flexibility and kink resistance, which are characteristics of coil structure, and in tensile strength, because the external surface of the coil layer and the internal surface of the first outer layer are in contact with each other in the slidable state, and also superior in resistance to cleavage because there is stress concentration partially in the outer layer particularly when tensile stress is applied and there is plastic deformation generated in the region. In particular, in a medical tube reinforced for example with a coil layer, the breaking strength of the coil itself is not considered to be so important because the tensile strength at break is relatively large, but the breaking strength of the region other than the coil is considered to be important. Thus, the present invention provides a technology very effective as a medical tube.

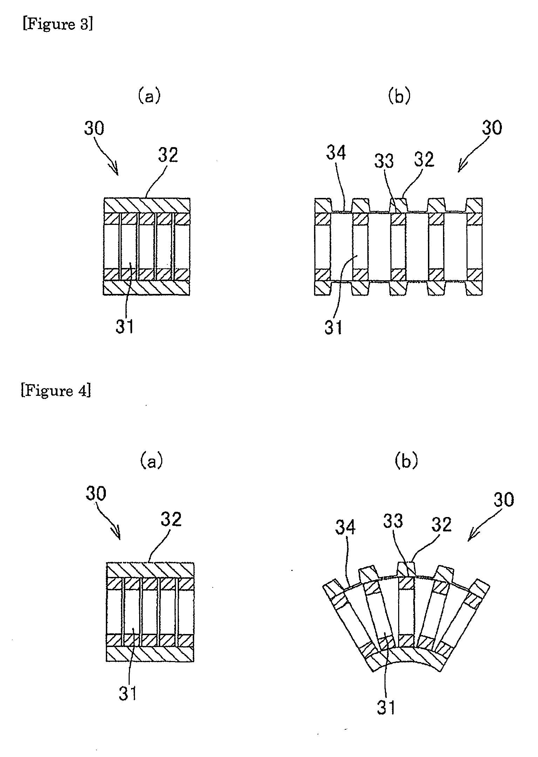

As described above, in the present invention, the state of "the external surface of the coil layer and the internal surface of the first outer layer are in contact with each other in a slidable state" means that the external surface of the coil layer and the internal surface of the first outer layer are in contact with each other without sliding from each other for example by frictional force when there is no stress, such as tensile or bending stress, applied to the medical tube, but the coil layer and the first outer layer can slide, independently of each other, when a stress is applied to the medical tube. The state will be described with reference to the typical examples shown in Figures 3 to 6, but is not limited to the states shown in these Figures. Figures 3 to 6 are simplified figures showing part of a medical tube.

Figures 3 and 4 are schematic views showing the cross section in the side wall direction of a conventional medical tube 30 before and after application of stress. In the conventional medical tube 30, a coil layer 31 and an outer layer 32 are fixed to each other inseparably. Specifically, the external surface of the coil layer 31 is connected to the inner surface of the outer layer 32 in the non-slidable state. In this case, in the medical tube 30 in the structure shown in Figure 3(a) or 4(a) when there is no stress applied, if the medical tube 30 is stretched in the lengthwise direction as stress is applied in the longitudinal direction (Figure 3(b)) or if bending stress is applied (Figure 4(b)), there is stress concentration in the unconnected regions 34 of the outer layer 32, causing plastic deformation or breakage as the coil is expanded because the coil layer 31 and the outer layer 32 are fixed to each other (in connected regions 33) and cannot slide independently of each other.

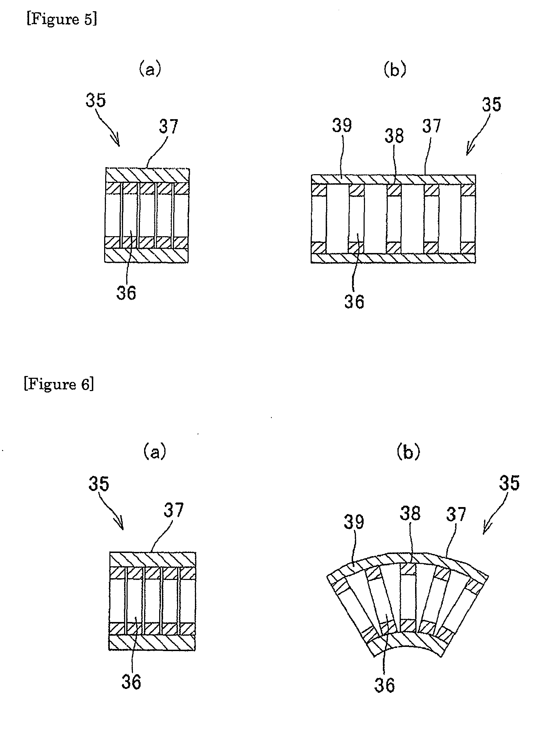

Figures 5 and 6 are schematic views illustrating the cross sections in the side wall direction of the medical tube according to the present invention 35 before and after application of stress. For simplification of the Figure, the first and second outer layers are drawn as integrated as an outer layer 37. The medical tube 35 according to the present invention has a coil layer 36 and an outer layer 37 in contact with each other in the slidable state. Specifically, the inner surface of the outer layer 37 and the external surface of the coil layer 36 are not fixed to each other in the inseparable state and can slide independently of each other when stress is applied. In this case, in the medical tube 35 in the structure shown in Figure 5(a) or 6(a) when there is no stress applied, it is possible to avoid stress concentration, reducing effectively plastic deformation and breakage of the outer layer 37, and thus of the medical tube, even if stress is applied in the longitudinal direction of the medical tube 35 (Figure 5(b)) or if bending stress is applied (Figure 6(b)), because the external surface of the coil layer 36 is not fixed to the inner surface of the outer layer 37 (in contact regions 38) and can slide independently of each other.

In addition, in the present invention, because the material constituting the second outer layer of the medical tube (first medical tube) has a melting point set to be lower than that of the material constituting the first outer layer, it is possible to form the second tube while preserving the tensile strength and to provide a catheter superior in insertion efficiency of guide wire, flexibility, kink resistance, and also tensile strength, for example if the medical tube is applied to a thrombus aspiration catheter having a guide wire lumen, particularly a rapid-exchange thrombus aspiration catheter.

Coil layers in various configurations are usable as the coil layers above, but a coil layer having at least one coil helically wound in one direction is particularly favorable, for prevention of deterioration in flexibility and kink resistance. The cross-sectional shape of the coil wire constituting the coil layer is not particularly limited, and wires in various shapes, such as flat wires, round wires, and irregular-shaped wires, can be used. Here, the flat wires have a cross-sectional shape in the direction perpendicular to the axial direction of the wire of rectangle or rounded rectangle, and include the so-called flat square wires.

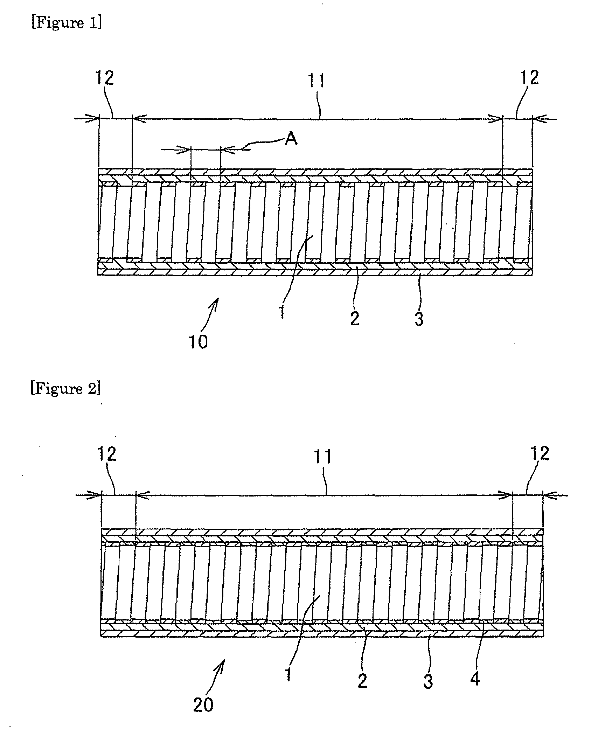

In addition, the pattern of coil winding is also not particularly limited, but a pattern having space between neighboring wires (hereinafter, referred to as pitch coil) is preferable, and in particular, the distance between the coil wires in the longitudinal direction of the medical tube is preferably the same as or larger than the width of the coil wire. In particular, catheters often demand flexibility in the distal region. It is possible to make a catheter flexible and kink resistant by using such a pitch coil. The term "pitch" means the length in the longitudinal direction between one point on the coil wire and another point of the wire after it is wound once in the circumferential direction (360 degrees) (indicated by A in Figure 1). More specifically, the pitch is the sum of the wire width and the distance between wires in the longitudinal direction. Thus, the phrase "the space between the coil wires is the same as or larger than the width of the coil wire," as used in the present invention means that, when the width of the wire is designated as t, the pitch is 2t or more.

Various materials including metals and resins can be used as the materials for the wire constituting the coil layer, but in particular, stainless steel or materials with high radiopacity, i.e., metals such as tungsten, platinum, iridium, and gold, are preferable. Among the materials above, stainless spring steel and tungsten, which are higher in tensile modulus of the wire, are preferable.

Examples of the resins constituting the first and second outer layers include, but are not particularly limited to, polyamides such as nylon 6, nylon 66, nylon 12, and polyamide elastomers; olefins such as polyethylene, polypropylene, polymethyl methacrylate, and modified polyolefins; polyesters such as polyethylene terephthalate, polybutylene terephthalate, and polyester elastomers; polyurethanes, polyurethane elastomers, polyether ether ketones, polyarylates, polyimides, polyamide-imides, the polymer blends and polymer alloys thereof, and the like. The resin material may contain, in addition to the polymerization aids that are used during polymerization, various additives such as contrast media, plasticizers, reinforcing agents, pigments and others. Preferable among these resins are polyamide elastomers and polyurethane elastomers that are higher in toughness under high bending stress. In addition, the first and second outer layers are preferably made of the same resin species, for improvement of the interfacial bonding strength.

In the present invention, the material constituting the second outer layer should have a melting point lower than that of the material constituting the first outer layer. It is possible in this case to fuse only the second outer layer, which is the layer outside of the first external surface, while leaving the first outer layer, which is the internal layer closer to the coil layer, unfused, by heating the tube at a temperature higher than the melting point of the material constituting the second outer layer but lower than the melting point of the material constituting the first outer layer. It is thus possible to bring the external surface of the coil layer and the internal surface of the first outer layer in contact with and fixed to each other in the slidable state and to preserve the high tensile strength of the medical tube. Thus, for example, when a second tube is to be formed in parallel with a first medical tube, it is possible to bond the first medical tube and the second tube by thermal bonding of the second outer layer and to assure the tensile strength with the first outer layer. It is possible in this way to produce a medical assembly and further a catheter. On the other hand, when the second outer layer has a melting point higher than that of the first outer layer, the first outer layer fuses when the second outer layer is melted, fixing the coil layer and the first outer layer in a non-slidable state and thus leading to deterioration in tensile strength because of the stress concentration described above.

In addition, when the second tube is connected to the distal end side or proximal end side of the first medical tube, it is possible to fuse the second outer layer without fusion of the first outer layer, and thus to connect it, while preserving the tensile strength (in particular, catheters often have rigidity inclination in the longitudinal direction, and it is possible in such a case to use the catheter effectively while preserving its tensile strength).

In the medical tube according to the present invention, the first outer layer is preferably oriented in the axial direction. Generally, resin and other materials have increased strength when they are oriented. It is possible to suppress the influence exerted by relaxation of resin orientation caused by heat treatment even when a medical tube is converted to a medical assembly or a catheter for example by thermal bonding, and thus to make the first outer layer retain relatively high strength.

In addition, the resin constituting the first outer layer is preferably a resin, particularly a thermoplastic resin, having a Shore D hardness of 50D or more because it gives a medical tube higher in tensile strength.

In the present invention, the external surface of the coil layer and the internal surface of the first outer layer should be in contact with and fixed to each other in a slidable state, and in particular, they are preferably brought into contact with and fixed to each other in the slidable state at rate of 0.5 or more with respect to the total length of the coil layer. For example, it is possible fix the external surface of the coil layer and the internal surface of the first outer layer to each other by a method such as thermal bonding, at the terminal of the medical tube or the like, but elongation of the fixing region leads to easier occurrence of the stress concentration, as described above, possibly prohibiting preservation of the tensile strength, and thus, it is needed to make the first outer layer and/or the second outer layer thicker. When the rate of the region in contact and fixed in the slidable state is 0.5 or more with respect to the total length of the coil layer, it is possible to overcome sufficiently the problems of reduction in diameter of the lumen of the medical tube by increase of the thickened region in the medical tube and deterioration in trackability due to increase of the external diameter. For the reasons above, the external surface of the coil layer and the first outer layer are preferably in contact with and fixed to each other in the slidable state over entire length of the coil layer, but it is also possible to fix only the coil end or part of the intermediate region, for example by thermal bonding, for fixation of the coil.

The medical tube according to the present invention may have an internal layer formed inside the coil layer additionally. Examples of the resins constituting the internal layer include, but are not particularly limited to, fluoroplastic resins such as polytetrafluoroethylene, polytetrafluoroethylene-perfluoroalkyl vinylether copolymers, polychlorotrifluoroethylene and tetrafluoroethylene-ethylene copolymers; polyolefins such as polypropylene and polyethylene; polyamides such as nylon 6, nylon 66, nylon 12, and polyamide elastomers; polyethylene terephthalate, polyester elastomers, polyurethanes, polyurethane-elastomers, polyimides and the like.

In the present invention, an intermediate layer of a flexible material may be formed in space between the coil wires constituting the coil layer. Such an intermediate layer, if formed, fills the space of the coil prevents generation of irregularity of the coil pitch during production (it is thus possible to form an intermediate layer only during production and remove it after production or leave it in the finished product as it is). In addition, the flexible material, if used, makes the medical tube more compatible with stress such as expanding or bending stress.

The intermediate layer may have any configuration if the advantageous effects are obtained, but when it is made of a resin, the resin constituting the intermediate layer preferably has a melting point higher than that of the resin constituting the second outer layer. If such a resin is used, in preparation of first and second outer layers after an intermediate layer is formed, the intermediate layer remains unfused when heated at a temperature higher than the melting point of the resin constituting the second outer layer and lower than that of the resin constituting the intermediate layer, making the handling of the product easier. As will be described below, when the first medical tube and the second tube are heated at a temperature similar to that above during thermal bonding, the intermediate layer and the first outer layer remain unfused and thus, it is possible to obtain a medical assembly or the like in which the external surface of the coil layer and the intermediate layer and the internal surface of the first outer layer are present in the slidable state. When a resin is used as the material constituting the intermediate layer, for example, a resin species similar to that for the first or second outer layer can be used.

Figure 1 is a side cross-sectional view illustrating an embodiment of the medical tube according to the present invention. The medical tube 10 in Figure 1 has a coil layer 1 and a first outer layer 2 and a second outer layer 3 formed outside the coil layer. It also has a region 11 where the external surface of the coil layer and the internal surface of the first outer layer are in contact with and fixed to each other in the slidable state and a region 12 where the external surface of the coil layer and the internal surface of the first outer layer are thermally bonded. In the present embodiment, the region 12 is prepared by filling the first outer layer 2 in the area between wires in the coil layer 1 by fusing the first outer layer 2.

Alternatively, Figure 2 is a side cross-sectional view illustrating another embodiment of the medical tube according to the present invention. In the configuration, an intermediate layer 4 is formed in the space between coil wires. More specifically, the medical tube 20 in Figure 2 has a coil layer 1, a first outer layer 2 formed outside the coil layer 1, a second outer layer 3 formed outside the outer layer 2, and an intermediate layer 4 formed between the coil wires constituting the coil layer 1. In Figure 2, the region where the external surfaces of the coil layer 1 and the intermediate layer 4 and the internal surface of the first outer layer are in contact with and fixed to each other in the slidable state is designated as region 11, while the region where the external surfaces of the coil layer 1 and the intermediate layer 4 and the internal surface of the first outer layer are thermally bonded is designated as region 12, and the same numerical codes are allocated to the components identical with those in Figure 1.

According to the present invention, it is possible to obtain a medical assembly by thermal bonding of a medical tube (first medical tube) with a second tube. In the medical assembly, the first medical tube and the second tube may be formed in any arrangement. The second tube may be formed in the proximal end region and/or the distal region of the first medical tube in parallel or concentrically with the first medical tube in the longitudinal direction. The second tube and the first medical tube may be formed in parallel or concentrically with it over the entire length.

The second tube may be in any structure and selected appropriately, for example, from single- and multi-lumen tubes according to the application of the medical assembly or the like. Various materials can be used as the materials constituting the second tube. However, the second tube contains at least partially a resin material having a melting point lower than that of the material constituting the first outer layer of the first medical tube. Specifically, a resin material having a melting point lower than that of the material constituting the first outer layer of first medical tube is used for the region of the second tube that fuses on the first medical tube. Accordingly, when the second tube is thermally bonded in parallel with the first medical tube, a resin material having melting point lower than that of the material constituting the first outer layer the first medical tube is used for at least part of the external surface of the second tube, preferably all of the region that fuses. On the other hand, when the first medical tube is thermally bonded concentrically inside the second tube, the resin material is used for at least part of the internal wall face of the second tube, preferably all of the region that fuses. Of course, the entire external surface of the second tube may be made of the particular resin material in the former case; the entire internal wall face may be made of the particular resin material in the latter case; and the entire second tube may be made of the particular resin material.

As described above, when the second tube has the configuration described above, it is possible, during thermal bonding of the first medical tube and the second tube, to fuse at a temperature higher than the melting point of the particular resin material of the second outer layer constituting the external surface of the first medical tube and the thermal bonding region of the second tube and lower than that of the resin constituting the first outer layer material (including the resin material for an intermediate layer, if present). As a result, it is possible to bond the first medical tube to the second tube by thermal bonding without fusion of the resin material constituting the first outer layer of the first medical tube, preserve the strength of the bonding region, make the external diameter smaller than that by use of an adhesive, and preserve the flexibility of the region. Further, since the resin material constituting the first outer layer of the first medical tube does not fuse, the first outer layer is neither bonded nor fixed to the coil layer in the inseparable state. It is thus possible to preserve the state in which the external surface of the coil layer and the internal surface of the first outer layer are in contact with and fixed to each other in the slidable state and to preserve the high tensile strength of the medical tube. Thus, the medical assembly according to the present invention is a product prepared from the first and second tube by thermal bonding, which retains the favorable properties of the first medical tube, and thus, a product extremely favorable as a medical device, which has additionally the favorable properties provided by thermal bonding.

In the present invention, a catheter is prepared by using the medical tube or the medical assembly. The catheter may be used as any catheter to be inserted into blood vessel, but catheters having a medical tube or a medical assembly in the distal region are preferable for demonstration of the favorable properties of the unit, and examples thereof include suction catheters, balloon catheters and the like. In particular, thrombus aspiration catheters, particularly rapid-exchange thrombus aspiration catheters, are favorable.

An embodiment of the thrombus aspiration catheter according to the present invention will be described briefly with reference to drawings.

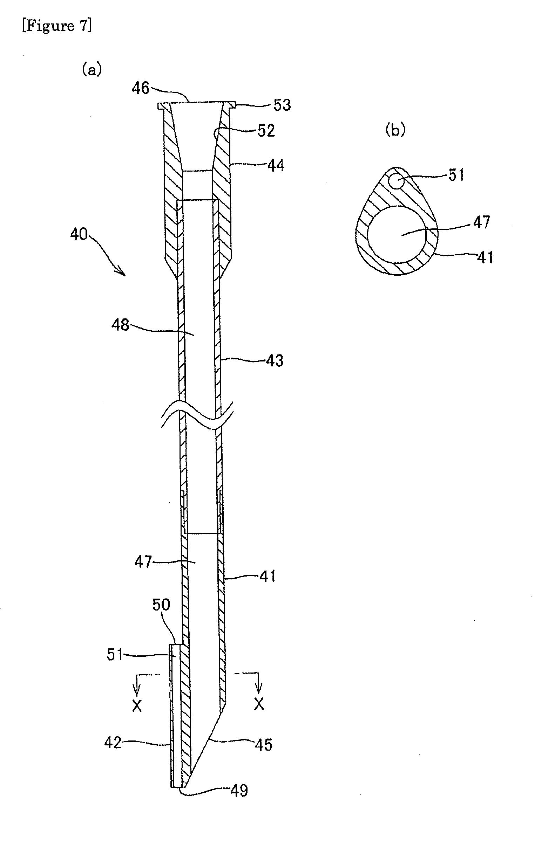

Figure 7(a) is a schematic view illustrating the cross section of a thrombus aspiration catheter 40 according to the present invention in the side wall direction. The thrombus aspiration catheter 40 is a rapid-exchange catheter having a first medical tube 41, a second tube 42, a proximal end side tube 43, and a hub 44 (in the embodiment, the region consisting of the first medical tube 41 and the second tube 42 is also a medical assembly according to the present invention). The first medical tube 41 has a multi-layered structure having a coil layer, a first outer layer and a second outer layer, as described above, but for simplification, the cross section of the first medical tube 41 is not drawn as such a multi-layered structure in Figure 7(a).

As shown in Figure 7(a), the thrombus aspiration catheter 40 has a second tube 42 formed in the distal region of the first medical tube 41 in parallel therewith and a proximal end side tube 43 and a hub 44 formed on the proximal end side thereof. In addition, the lumen 47 in the first medical tube 41 and the lumen 48 in the proximal end side tube 43 extend, as connected, over the entire range from the distal end opening 45 of the first medical tube to the proximal end side opening 46 of the hub 44, forming a lumen for aspiration of thrombus in blood vessel. A tube higher in flexural rigidity than the first medical tube (or medical assembly) is preferably used as the proximal end side tube 43, for improvement of the operability of the suction catheter 40. The tube is, for example, a medical tube according to the present invention having higher flexural rigidity.

The second tube 42 has a lumen 51 extending from the distal end side opening 49 to the proximal end side opening 50, forming a lumen for insertion of a guide wire not shown in the Figure. Alternatively, the hub 44 has a tapered lumen 52, and the proximal end-sided external surface region has a protrusion 53 in the configuration allowing luer-lock connection with a catheter or the like not shown in the Figure for suction of thrombus. A removable core wire may be placed additionally in the lumen for suction of thrombus for prevention of kink of the thrombus aspiration catheter 40.

Figure 7(b) is a schematic view illustrating the X-X cross section in Figure 7(a). For simplification, the cross section of the first medical tube 41 is not drawn as a multi-layered structure also in this figure. As described above, the first medical tube 41 and the second tube 42 are thermally bonded to each other, and when the thermal bonding is carried out for example by the method described below in Examples, the resin melts, forming a mostly circular cross section as shown in Fig. 7(b).

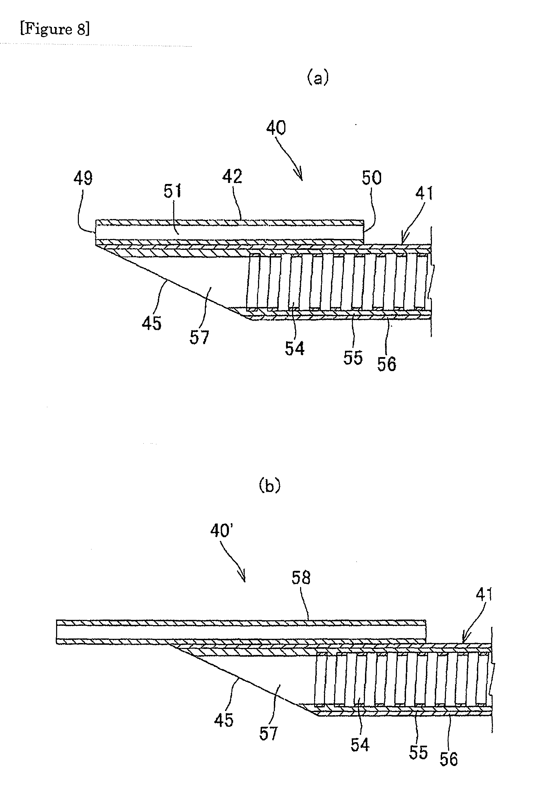

Figure 8(a) is an expanded schematic view of the cross section of the distal region of the thrombus aspiration catheter 40 in the side wall direction, specifically showing the configuration of the first medical tube 41. In this embodiment, the distal end opening 45 has a cross section tilted to the axial direction of the first medical tube 41, leading to increase in the sectional area of the distal end opening 45. In addition, the most distal region 57 is made only of a flexible material and does not contain the coil layer 54, for prevention of the damage of blood vessel and others by the distal region. The flexible material for use may be the resin material constituting the first outer layer 55 and/or the second outer layer 56 or a resin material other than that. In addition, the most distal region 57 may be formed by bonding a tube separated prepared, or a first outer layer and/or a second outer layer previously prepared slightly longer may be used as it is.

In the embodiment above, the first outer layer 55 and/or the flexible material present between the coil wires constituting the coil layer 54 are placed in the distal region of the coil layer 54 for fixation of the coil layer 54 and thus for prevention of the coil layer 54 protruding from the distal region. The configuration of the region of the coil layer 54 is not limited to that described above, if the protrusion-preventing effect is obtained. For convenience of operation of the thrombus aspiration catheter, the second tube 58 may be protrude from the distal end side out of the distal end opening 45 of the first medical tube 41, as shown in Figure 8(b).

As for other detailed configuration, the configuration for example described in JP-A No. 2004-65326 or WO 2005-44359 pamphlet may be applied, as needed, in the range that does not impair the advantageous effects of the present invention.

The method of producing the medical assembly according to the present invention will be described briefly.
First, a first medical tube is prepared. A coil for the coil layer is covered with a laminate tube of first and second outer layers previously prepared or with a tube for the first outer layer and a tube for the second outer layer sequentially. It is then preferable to use a core material that can be inserted into the coil from the viewpoint of processability and for prevention of coil breakage. After coating, as needed, the coil layer and the two-layer tube may be bonded to each other by thermal bonding, as part of the two-layered tube in the entire length is fused under heat.

If an intermediate layer is formed, for example, a core material having a diameter similar to the inner diameter of the coil forming the coil layer is inserted into the coil, and the core layer is covered with a tube made of a resin forming the intermediate layer. It is additionally coated with a heat-shrinkable tube from outside, and the composite is heated at a temperature higher than the melting point of the resin constituting the tube for a particular time, forming the intermediate layer, for reduction of the diameter of the heat-shrinkable tube and placement of the resin forming the intermediate layer in the area between coil wires by thermal bonding. The heating method for use may be any known method. After the heat-shrinkable tube is cooled, it is removed. A coil layer having an intermediate layer is formed in this way, and a first medical tube is prepared therefrom similarly to above.

The first medical tube prepared as described above is then thermally bonded to a second tube. In the present invention, the first medical tube is thermally bonded to a second tube containing at least partially a resin material having a melting point lower than that of the material constituting the first outer layer of the first medical tube, to give a medical assembly. The region of the resin material in the second tube is then thermally bonded to the first medical tube, as the medical assembly is heated at a temperature lower than the melting point of the material constituting the first outer layer of the first medical tube and higher than that of the material constituting the second outer layer. When an intermediate layer of resin is formed on the first medical tube, the temperature for fusion of a particular region of the second tube to the first medical tube is preferably a temperature lower than the melting point of the resin constituting the intermediate layer for prevention of thermal bonding between the intermediate layer and the first outer layer.

Specifically, when the first medical tube thus obtained and the particular second tube above are thermally bonded as aligned in parallel with each other, the first medical tube having a core material inserted therein and the second tube containing at least partially a particular resin material in the external surface and also having a core material inserted therein are aligned in parallel with each other, and then, a heat-shrinkable tube for example is placed around these two tubes where they are to be bonded. The region to be bonded and covered with the heat-shrinkable tube is then heated at the temperature described above for reduction of the diameter of the heat-shrinkable tube and simultaneously for fusion and thermal bonding of the particular resin placed in the bonding region between the second outer layer of the first medical tube and the second tube. Subsequent removal of the heat-shrinkable tube after cooling gives a double-lumen medical assembly having the first medical tube and the second tube aligned in parallel. The heating method for use is not limited to the method of using a heat-shrinkable tube and may be any known method, and the heating means for use may be any known apparatus such as oven.

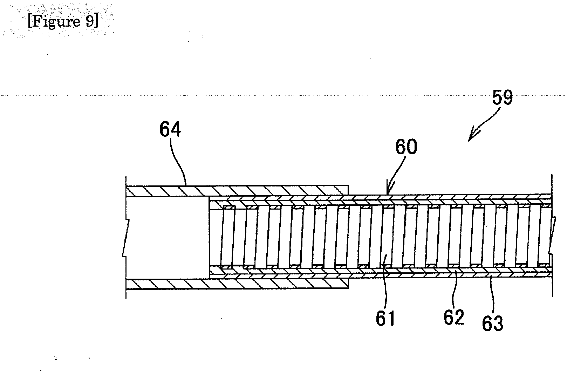

Alternatively, when a first medical tube and a particular second tube are thermally bonded concentrically, the first medical tube having a core material inserted therein is covered concentrically with a second tube with a desired length, having the particular resin material constituting the thermal bonding region of the internal wall face. For example, when a heat-shrinkable tube is used, the second tube is covered with a heat-shrinkable tube and heated similarly to above to give a medical assembly having a second tube thermally bonded concentrically onto part of the external surface of the first medical tube. Figure 9 is a schematic side crosssectional view illustrating the structure of the bonding region of the medical assembly. As shown in the Figure, the first medical tube 60 has a coil layer 61, a first outer layer 62, and a second outer layer 63, and in the medical assembly 59, the second outer layer 63 constituting the external surface region of the first medical tube 60 and the internal wall face of a second tube 64 are thermally bonded to each other concentrically.

### Examples

Hereinafter, the present invention will be described in detail with reference to Examples, but it should be understood that the present invention is not limited to the following Examples.

### (Example 1)

A stainless steel core material having a diameter of 1.00 mm and a length of 500 mm was inserted into a coil having an inner diameter of 1.00 mm, a wire-wire distance of 0.05 mm (pitch: 0.15 mm), and a length of 400 mm, which is made of a flat stainless steel wire (thickness: 0.02 mm, width: 0.10 mm). The coil having the stainless steel core material inserted therein was covered carefully with a two-layer tube (inner diameter: 1.04 mm, external diameter: 1.18 mm, length: 300 mm) consisting of an internal layer (first outer layer) having a thickness of 0.05 mm and made of a polyamide elastomer (Shore D hardness: 70D, melting point 174°C) and an external layer (second outer layer) having a thickness of 0.02 mm and made of a polyamide elastomer (Shore D hardness: 35D, melting point: 152°C), and the stainless steel core material having a diameter of 1.00 mm was removed, to give a first medical tube having an inner diameter of 1.00 mm and an external diameter of 1.18 mm.

Separately, a second tube (internal diameter: 0.41 mm, external diameter: 0.56 mm, length: 150 mm) of a polyamide elastomer (Shore D hardness: 55D, melting point: 168°C), having a stainless steel core material having a diameter of 0.40 mm inserted therein was placed in parallel with the first medical tube in the region thereof of 0 to 150 mm from the terminal of the first medical tube (containing the core material inserted therein again), and the first medical tube and the second tube were covered with a heat-shrinkable tube and heated in an oven adjusted to 170°C for 2 minutes. After taken out of the oven, the heat-shrinkable tube was removed and the stainless steel core material having a diameter of 1.00 mm and the stainless steel core material having a diameter of 0.40 mm were both removed to give a medical assembly having a 150 mm region where the first and second tubes are thermally bonded to each other in parallel and a 150 mm region only of the first medical tube.

### (Kink resistance)

The first medical tube was left straight; two points thereof separated by 50 mm in the longitudinal direction were held by hands; and the hands were pushed inward on a straight line to a distance of 10 mm, allowing the tube to bend. The tube was resistant to collapse or separation of outer layer and showed favorable kink resistance. Subsequently, the region of the medical assembly where the second tube is thermally bonded in parallel was also evaluated in a kink resistance test similar to that for the first medical tube, and the medical assembly was resistant to collapse or separation of outer layer and showed favorable kink resistance.

### (Tensile strength)

The tensile strength (strength when the medical tube is broken) of the first medical tube was determined by using a tensile compression tester (SHIMADZU CORPORATION) under the condition of a chuck distance of 50 mm and a stress rate of 1000 mm/min. The strength when the outer layer is broken was found to be sufficiently high at 14 N. Then, the tensile strength of the region of the medical assembly where the second tube is thermally bonded in parallel was also determined similarly to the first medical tube. The strength when the outer layer is broken was found to be sufficiently high at 15 N.

### (Example 2)

A stainless steel core material having a diameter of 1.25 mm and a length of 500 mm was inserted into a coil having an inner diameter of 1.25 mm, a wire-wire distance of 0.08 mm (pitch: 0.18 mm), and a length of 400 mm, which is made of a flat stainless steel wire (thickness: 0.05 mm, width: 0.10 mm). The coil having the stainless steel core material inserted therein was covered carefully with a two-layer tube (inner diameter: 1.35 mm, external diameter: 1.47 mm, length: 300 mm) consisting of an internal layer (first outer layer) having a thickness of 0.04 mm and made of a polyurethane elastomer (Shore D hardness: 68D, melting point: 182°C) and an external layer (second outer layer) having a thickness of 0.02 mm and made of a polyurethane elastomer (Shore A hardness: 85A, melting point: 163°C), and the stainless steel core material having a diameter of 1.25 mm was removed, to give a first medical tube having an inner diameter of 1.25 mm and an external diameter of 1.47 mm.

Separately, a second tube (internal diameter: 0.41 mm, external diameter: 0.56 mm, length: 150 mm) of a polyurethane elastomer (Shore D hardness: 51D, melting point: 167°C), having a stainless steel core material having a diameter of 0.40 mm inserted therein was placed in parallel with the first medical tube in the region thereof of 0 to 150 mm from the terminal of the first medical tube (containing the core material inserted therein again), and the first medical tube and the second tube were covered with a heat-shrinkable tube and heated in an oven adjusted to 174°C for 2 minutes. After taken out of the oven, the heat-shrinkable tube was removed and the stainless steel core material having a diameter of 1.25 mm and the stainless steel core material having a diameter of 0.40 mm were both removed, to give a medical assembly having a 150 mm region where the first and second tubes are thermally bonded to each other in parallel and a 150 mm region only of the first medical tube.

The kink resistance and the tensile strength respectively of the medical tube and the medical assembly were determined by methods similar to those in Example 1.

Both the medical tube and the medical assembly were resistant to collapse or separation of outer layer and showed favorable kink resistance.

In measurement of the tensile strength, both the medical tube and the medical assembly showed sufficiently high strength when the outer layer is broken, respectively at 16 N and 17 N.

### (Example 3)

A stainless steel core material having a diameter of 1.00 mm and a length of 500 mm was inserted into a coil having an inner diameter of 1.00 mm, a wire-wire distance of 0.10 mm (pitch: 0.20 mm), and a length of 400 mm, which is made of a flat stainless steel wire (thickness: 0.03 mm, width: 0.10 mm). The coil having the stainless steel core material inserted therein was covered carefully with a two-layer tube (inner diameter: 1.06 mm, external diameter: 1.16 mm, length: 300 mm) consisting of an internal layer (first outer layer) having a thickness of 0.04 mm and made of a polyamide elastomer (Shore D hardness: 70D, melting point: 174°C) and an external layer (second outer layer) having a thickness of 0.01 mm and made of a polyamide elastomer (Shore D hardness: 35D, melting point: 152°C), and the stainless steel core material having a diameter of 1.00 mm was removed, to give a first medical tube having an inner diameter of 1.00 mm and an external diameter of 1.16 mm. Then, a medical assembly having a 150 mm region only of the first medical tube and a 150 mm region where the first and second tubes are thermally bonded in parallel, was obtained by a method similar to that in Example 1.

The kink resistance and the tensile strength respectively of the medical tube and the medical assembly were determined by methods similar to those in Example 1.

Both the medical tube and the medical assembly were resistant to collapse or separation of outer layer and showed favorable kink resistance.

In measurement of the tensile strength, both the medical tube and the medical assembly showed sufficiently high strength when the outer layer is broken, respectively at 12 N and 13 N.

### (Example 4)

A stainless steel core material having a diameter of 1.00 mm and a length of 500 mm was inserted into a coil of having an inner diameter of 1.00 mm, a wire-wire distance of 0.05 mm (pitch: 0.15 mm) and a length of 400 mm, which is made of a flat stainless steel wire (thickness: 0.03 mm, width: 0.10 mm). The coil having the stainless steel core material inserted therein was covered with an intermediate layer (inner diameter: 1.16 mm, external diameter: 1.20 mm, length: 300 mm) made of a polyamide elastomer (Shore D hardness: 63D, melting point: 172°C). A heat-shrinkable tube was placed further on the intermediate layer and heated in an oven adjusted to 200°C for 2 minutes. After taken out of the oven, the heat-shrinkable tube was removed; and a two-layer tube (inner diameter: 1.06 mm, external diameter: 1.20 mm, length: 300 mm) consisting of an internal layer (first outer layer) having a thickness of 0.05 mm and made of a polyamide elastomer (Shore D hardness: 70D, melting point: 174°C) and an external layer (second outer layer) having a thickness of 0.02 mm and made of a polyamide elastomer (Shore D hardness: 35D, melting point: 152°C) was placed over the intermediate layer; and the stainless steel core material having a diameter of 1.00 mm was removed, to give a first medical tube having an inner diameter of 1.00 mm and an external diameter of 1.20 mm. Then, a medical assembly having a 150 mm region only of the first medical tube and a 150 mm region where the first and second tubes are thermally bonded in parallel, was obtained by a method similar to that in Example 1.

The kink resistance and the tensile strength respectively of the medical tube and the medical assembly were determined by methods similar to those in Example 1.

Both the medical tube and the medical assembly were resistant to collapse or separation of outer layer and showed favorable kink resistance.

In measurement of the tensile strength, both the medical tube and the medical assembly showed sufficiently high strength when the outer layer is broken, respectively at 15 N and 16 N.

### (Comparative Example 1)

A stainless steel core material having a diameter of 1.00 mm and a length of 500 mm was inserted into a coil of having an inner diameter of 1.00 mm, a wire-wire distance of 0.05 mm (pitch: 0.15 mm) and a length of 400 mm, which is made of a flat stainless steel wire (thickness: 0.02 mm, width: 0.10 mm). The coil having the stainless steel core material inserted therein was covered with a two-layer tube (inner diameter: 1.04 mm, external diameter: 1.18 mm, length: 300 mm) consisting of an internal layer (first outer layer) having a thickness of 0.02 mm and made of a polyamide elastomer (Shore D hardness: 35D, melting point: 152°C) and an external layer (second outer layer) having a thickness of 0.05 mm and made of a polyamide elastomer (Shore D hardness: 70D, melting point: 174°C), and the stainless steel core material having a diameter of 1.00 mm was removed, to give a first medical tube having an inner diameter of 1.00 mm and an external diameter of 1.18 mm. A second tube (internal diameter: 0.41 mm, external diameter: 0.56 mm, length: 150 mm) of a polyamide elastomer (Shore D hardness: 55D, melting point: 168°C), having a stainless steel core material having a diameter of 0.40 mm inserted therein was placed in parallel with the first medical tube in the region thereof of 0 to 150 mm from the terminal of the first medical tube, and the first medical tube and the second tube were covered with a heat-shrinkable tube and heated in an oven adjusted to 170°C for 2 minutes. After taken out of the oven and separation of the heat-shrinkable tube, the thermal bonding between the first medical tube and the second tube was low and thus these tubes are easily separated, prohibiting production of a favorable medical assembly. Subsequently, a first medical tube prepared by a similar method was heated in an oven adjusted to 178°C for 2 minutes. After taken out of the oven and separation of the heat-shrinkable tube, the stainless steel core material having a diameter of 1.00 mm and the stainless steel core material having a diameter of 0.40 mm were both removed, to give a medical assembly having a 150 mm region where the first and second tubes are thermally bonded in parallel and a 150 mm region only of the first medical tube.

Both the medical tube and the medical assembly were resistant to collapse or separation of outer layer and showed favorable kink resistance.

In measurement of the tensile strength, the medical tube had a sufficient tensile strength (strength when the outer layer is broken) of 14 N, but the medical assembly was broken at a lower tensile strength of 7N.

### (Comparative Example 2)

A stainless steel core material having a diameter of 1.00 mm and a length of 500 mm was inserted into a coil of having an inner diameter of 1.00 mm, a wire-wire distance of 0.05 mm (pitch: 0.15 mm) and a length of 400 mm, which is made of a flat stainless steel wire (thickness: 0.02 mm, width: 0.10 mm). The coil having the stainless steel core material inserted therein was covered with a single layer tube (inner diameter 1.04mm, external diameter 1.14 mm, length 300 mm) having a thickness of 0.05 mm and made of a polyamide elastomer (Shore D hardness: 70D, melting point: 174°C), and the stainless steel core material having a diameter of 1.00 mm was removed, to give a first medical tube having an inner diameter of 1.00 mm and an external diameter of 1.14 mm. A second tube (internal diameter: 0.41 mm, external diameter: 0.56 mm, length: 150 mm) of a polyamide elastomer (Shore D hardness: 55D, melting point: 168°C), having a stainless steel core material having a diameter of 0.40 mm inserted therein was placed in parallel with the first medical tube in the region thereof of 0 to 150 mm from the terminal of the first medical tube, and the first medical tube and the second tube were covered with a heat-shrinkable tube and heated in an oven adjusted to 170°C for 2 minutes. After taken out of the oven and separation of the heat-shrinkable tube, the thermal bonding between the first medical tube and the second tube was low and thus these tubes are easily separated, prohibiting production of a favorable medical assembly. Subsequently, a first medical tube prepared by a similar method was heated in an oven adjusted to 178°C for 2 minutes. After taken out of the oven and separation of the heat-shrinkable tube, the stainless steel core material having a diameter of 1.00 mm and the stainless steel core material having a diameter of 0.40 mm were both removed, to give a medical assembly having a 150 mm region where the first and second tubes are thermally bonded in parallel and a 150 mm region only of the first medical tube.

The medical tube and the medical assembly were both broken when the hands holding them were pushed inward to a distance of 50 mm to 30 mm, indicating their low kink resistance.

In measurement of the tensile strength, the medical tube had a sufficient tensile strength (strength when the outer layer is broken) of 13 N, but the medical assembly was broken at a low tensile strength of 6N.

### (Example 5)

A first medical tube was prepared in a manner similar to Example 1. Separately, a second tube (internal diameter: 1.25 mm, external diameter 1.50 mm, length 100 mm) of a polyamide elastomer (Shore D hardness: 55D, melting point: 168°C) was placed coaxially with the first medical tube in the region thereof of 0 to 50 mm from the terminal of the first medical tube, so that the external surface of the first tube becomes in contact with the internal surface of the second tube at least partially in the peripheral region. A stainless steel core material having a diameter of 1.00 mm was inserted into the first tube and second tubes as it penetrates therein, and a heat-shrinkable tube was wrapped around the region where the first medical tube and the second tube are formed coaxially and heated in an oven adjusted to 170°C for 2 minutes. After taken out of the oven and separation of the heat-shrinkable tube, the stainless steel core material having a diameter of 1.00 mm was removed, to give a medical assembly having a 50 mm region where the first and the second tubes are thermally bonded to each other.

The kink resistance and the tensile strength respectively of the medical tube and the medical assembly were determined by methods similar to those in Example 1.

Both the medical tube and the medical assembly were resistant to collapse or separation of outer layer and showed favorable kink resistance.

In measurement of the tensile strength, both the medical tube and the medical assembly showed sufficiently high strength when the outer layer is broken, respectively at 16 N and 15 N.

### Reference Signs List

- 1: Coil layer
- 2: First outer layer
- 3: Second outer layer
- 4: Intermediate layer
- 10: First medical tube
- 11: Region where the external surface of the coil layer and the internal surface of the first outer layer are in contact with and fixed to each other in a slidable state
- 12: Region where the external surface of the coil layer and the internal surface of the first outer layer are thermally bonded to each other
- 20: First medical tube
- 30: Conventional medical tube
- 31: Coil layer
- 32: Outer layer
- 33: Connected region
- 34: Unconnected region
- 35: Medical tube
- 36: Coil layer
- 37: Outer layer
- 38: Contact region
- 39: Non-contact region
- 40: Thrombus aspiration catheter
- 41: First medical tube
- 42: Second tube
- 43: Proximal end side tube
- 44: Hub
- 45: Distal end opening
- 46: Proximal end opening
- 47, 48: Lumen
- 49: Distal end side opening
- 50: Proximal end side opening
- 51: Lumen
- 52: Tapered lumen
- 53: Protrusion
- 54, 61: Coil layer
- 55, 62: First outer layer
- 56, 63: Second outer layer
- 57: Most distal region
- 58, 64: Second tube
- 59: Medical assembly
- A: Coil pitch length

## Claims

1. A medical tube comprising at least one coil layer, a first outer layer formed outside the coil layer, and a second outer layer formed outside the first outer layer, wherein the material constituting the second outer layer has a melting point lower than that of the material constituting the first outer layer and the external surface of the coil layer and the internal surface of the first outer layer are in contact with each other in a slidable state.

2. The medical tube according to Claim 1, wherein the external surface of the coil layer and the internal surface of the first outer layer are in contact with each other in a slidable state, at a rate of at least of 0.5 or more with respect to the entire coil layer.

3. The medical tube according to Claim 1 or 2, wherein the first outer layer is made of a thermoplastic resin having a Shore D hardness of 50 D or more.

4. The medical tube according to any one of Claims 1 to 3, wherein the coil layer has an internal layer inside.

5. The medical tube according to any one of Claims 1 to 4, wherein the coil layer is configured with a coil having space between neighboring wires.

6. The medical tube according to Claim 5, wherein the distance between the coil wires in the longitudinal direction of the medical tube is the same as or longer than the width of the coil wire.

7. The medical tube according to any one of Claims 1 to 6, wherein the first outer layer is oriented in the axial direction.

8. The medical tube according to any one of Claims 5 to 7, wherein an intermediate layer of a flexible material is formed in space between the coil wires.

9. A medical assembly, comprising the medical tube (first medical tube) according to any one of Claims 1 to 8 and a second tube containing at least partially a resin material having a melting point lower than that of the material constituting the first outer layer of the first medical tube, wherein the region of the resin material in the second tube is thermally bonded to the first tube.

10. The medical assembly according to Claim 9, wherein the region of the resin material in the second tube is placed on the external surface of the second tube.

11. A catheter, comprising the medical tube according to any one of Claims 1 to 8 or the medical assembly according to Claim 9 or 10.

12. The catheter according to Claim 11, wherein the medical tube or the medical assembly is formed in the distal region thereof.

13. The catheter according to Claim 11 or 12, wherein the catheter is a thrombus aspiration catheter.

14. A method of producing a medical assembly containing the medical tube (first medical tube) according to any one of Claims 1 to 8 and a second tube containing at least partially a resin material having a melting point lower than that of the material constituting the first outer layer of the first medical tube, **characterized in that** the region of the resin material in the second tube is thermally bonded to the first tube, at a temperature lower than the melting point of the material constituting the first outer layer of the first medical tube and higher than the melting point of the material constituting the second outer layer.

15. The method of producing a medical assembly according to Claim 14, wherein the region of the resin material in the second tube, which is placed on the external surface of the second tube, is thermally bonded to the first tube.
